# EUROPEAN PATENT APPLICATION

(11) **EP 1 493 447 A2**
(43) Date of publication of application: **05.01.2005**
(21) Application number: 03731653.6
(22) Date of filing: 22.01.2003
(51) Int. Cl.: A61K 39/29, A61K 39/295

(54) **HEPATITIS B VIRUS SURFACE ANTIGEN AS A MUCOSAL IMMUNOSTIMULATOR AND THE RESULTING FORMULATIONS**

(30) Priority: 24.01.2002 CU 1902
(71) Applicant: CENTRO DE INGENIERIA GENETICA Y BIOTECNOLOGIA, Ciudad de La Habana 10600 (CU)
(72) Inventor: AGUILAR RUBIDO, Julio, César, 32200 La Habana (CU); ALEMAN ZALDIVAR, Regis, Calle 47 No 1122 e/, 10600 Ciudad Habana (CU); LOBAINA MATO, Yadira, Calle Palmar No. 12813 e/, Municipio Cerro, 13400 C. Habana (CU); PAJON FEYT, Rolando, 32400 La Habana (CU); MUZIO GONZALEZ, Verena, Lucila, 10600 Ciudad de La Habana (CU); GUILLEN NIETO, Gerardo, Enrique, 10400 Ciudad de La Habana (CU); ALVAREZ OBREGON, Julio César, Calle 186 No 3115 e, 10600 Ciudad de La Habana (CU); GARCIA GONZALEZ, Daymir, Ave 35 e/ 234 y 236 Edif, 13600 Ciudad de La Habana (CU); IGLESIAS PEREZ, Enrique, Edificio 25 Apto. 103, 11700 Ciudad de La Habana (CU); SARDINAS GARCIA.G., Jorge No 18 e/, Municipio 10 de Oct., 10500 Ciudad de La (CU); HARDY RANDO, Eugenio, Calle 184 No 3112 e/, 10600 Ciudad de La Habana (CU); PENTON ARIAS, Eduardo, Calle 31 No. 18207 e/, 10600 Ciudad de LaH abana (CU); URQUIZA NOA, Dioslaida, Calle 186 No 3115 Apto 11H, 10600 Ciudad de LaH abana (CU)
(74) Representative: Winckels, Johannes Hubertus F.
(86) International application number: PCT/CU2003/000001
(87) International publication number: WO 2003/061692

(57) **Abstract**

The invention relates to a mucosal surface antigen which is used to promote and increase in the immune response against co-administered antigens in the formulations out line in the invention.

Said novel formulations are obtained from the dual use of the surface antigen as an immunostimulatory agent and, at the same time, as a vaccine antigen. In this way it is possible to obtain multiple formulations of the hepatitis B surface antigen and heterologous antigens, with immunogenicity levels similar to those obtained following parenteral administration and with a reduction in components that can dispense with the use of nasal adjuvants, thereby converting same antigens into elements that can promote an increase in the response to the other co-administered antigens.

Said novel use of the hepatitis B virus surface antigen and the resulting antigen formulations can be used in the pharmaceutical industry as therapeutic and preventive vaccine formulations.

## Description

### Technical branch

The current invention is concerned with the field of vaccine development, specifically with the development of immunoenhancers and vaccine formulations resulting from their use.

The technical aim of this invention is to favor an enhancement in the immune response against antigens administered in nasal formulations and to develop new formulations for vaccine use by this route.

This invention is also related to the obtainment of multivalent vaccine formulations for nasal administration, having the hepatitis B virus surface antigen as the main antigen that is able to enhance the immunogenicity of the co-administered antigens in the formulations expressed in this invention.

The current invention has multivalent formulations containing HBsAg and other antigens, including soluble antigens as toxoids and their conjugates, and inactivated or attenuated vaccine microorganisms. Other antigens commonly used in commercial immunization have been included in this type of formulation with identical results, and immunogenicity levels, both humoral and cellular; these are very similar to those obtained after parenteral administration in the conventional formulations. A unique characteristic of this type of mucosal formulation is the induction of a strong response at mucosa, which is also an additional advantage. Another important aspect is the economy of components of these formulations since the antigens themselves may favor an increase in response against the other co-administered antigens, allowing the use of non nasal adjuvants.

### Previous technique

The generation of strong immune responses against antigens inoculated via mucosa is one of the current challenges the research in the vaccine field. It has been demonstrated that a strong local response correlates with protection against pathogens found or entering through the mucosal surfaces (American Academy of Pediatrics. Cholera. Report of the Committee on Infectious Diseases. American Academy of Pediatrics, Elk Grove Village, 1991; IL, 170).

The mucosal surface of the gastrointestinal, respiratory and genitourinary tracts has a surface area of at least 400 m² (McKenzie S J, Halsey J A. 1984; J. Immunol., 53:1818). On the other hand, a large specialized immune system protects the mucosal surfaces. In a healthy adult individual this local immune system has at least 80% of all lymphocytes, which can be located at this site or circulating through several mucosa-associated lymphoid tissues (Report of the Expert Panel VI: Concerted efforts in the field of mucosal immunology. 1996. Vaccine;14: 644).

The mucosal immune system is divided into local inductor sites, called organized mucosa-associated lymphoid tissues (O-MALT), and effector sites (Kraehenbuhl J P and Neutra M N. 1992. Physiol. Rev.;72:853). Most of the studies on O-MALT have been carried out in gastrointestinal-associated lymphoid tissues (GALT) such as Peyer's patches (PP), appendix, and isolated lymphatic nodes that are abundant at the rectum. PP is a good model of the functioning of these tissues, although the differences with GALT a nasal-associated lymphoid tissue (NALT) (Walker R I. 1994. Vaccine.;4:387) should be taken into account.

In the respiratory tract, the epithelium can be pseudostratified or simple. In bronchi, simple epithelium zone, intercellular spaces are sealed by tight bindings and the main mechanism of antigen uptake is through M cells. The stratified epithelium predominates at the tonsil, where the mechanism of antigen uptake is closely related to a net of macrophages and mobile dendritic cells arising from the bone marrow, of up to 700 cells per mm². These cells are able to migrate to the O-MALT and to lymphnodes, presenting the processed antigen that underwent phagocytosis at the tonsil surface. This is the main mechanism of antigen presentation by MHC-II in the respiratory tract under normal conditions (Neutra R M., Pringault E and Kraehenbuhl J P. 1996. Annu. Rev. Immunol. 14: 275).

After processing and presenting the antigen at inductor sites, the stimulated B and T lymphocytes leave the inductor sites through efferent ducts and enter the bloodstream via the thoracic duct, reaching the effector sites (glandular tissues, lamina propia of gastrointestinal tract mucosa, respiratory and genitourinary tracts where they are selectively retained. In these effector sites, B cells continue proliferating and are differentiated into secretory IgA plasmatic cells, with the subsequent production of IgA antibodies in external secretions. This system of cellular distribution that was previously described is called common mucosal immune system (Walker R I. 1994. Vaccine.;4:387).

Mucosal inoculation of vaccine antigens offers many advantages with respect to vaccines administered by the parenteral route, such as an increase in safety and the minimization of adverse effects (Editorial. Typhoid vaccination: Weighing the options. 1992. Lancet: 340-341; Redhead K and Griffiths E. 1990. Curr. Opin. Infect. Dis. 3:380), the qualified personnel can be reduced and the logistic of vaccination simplified, as well as an increase in the effectiveness of vaccination to the elderly and newborn children. It has been confirmed that the human systemic immune system is depressed with age, whereas studies in mice have shown no depression on aging (Bergman K-C and Waldman R H. Rev. 1988. Infect. Dis. 10: 939; Szewczuk M R, Campbell R J and Jung L K. 1981. J. Immunol. 126: 2200). In newborns the persistence of maternal antibodies interferes with vaccines administered parenterally, which has been problem in the reduction of vaccination age (Szewczuk M R, Campbell R J and Jung L K. 1981. J. Immunol. 126: 2200; Weiss R. 1992. Science. 258: 546).

Mucosal immunization can also facilitate the eradication of certain diseases caused by pathogens that are colonizing the mucosal surfaces asymptomatically (Kraehenbuhl J P and Neutra M N. 1992. Physiol Rev.72:853). This is due to the fact that this kind of immunization can not only generate systemic responses, but also mucosal responses, which is not attained with inoculations through the parenteral route.

Despite the previously mentioned advantages the amount of antigen required for mucosal immunization can be higher than that needed for parenteral immunization probably due to several factors such as the relative ineffectiveness of the uptake of the intact antigen by the mucosal lymphoid tissue, acidic and proteolitic barriers, intestinal peristaltism, among others. That is why it is necessary to develop adjuvants or adjuvanticity strategies for mucosal use (Faden H, *et al*. 1990. J. Infect. Dis. 162: 1291; Shahin RD, *et al*. 1990. Infect. Immun. 58: 4063; O'Hagan D T. 1990. Curr Opin Infect Dis. 3: 393).

The pure, recombinant or synthetic antigens from new vaccine generations have been safer than those from the organism they were obtained. However, they are less immunogenic (Alving C R, 1992. AIDS Research and Human Retroviruses.8:1427). Hence, finding new adjuvants is a need in the vaccine field.

Adjuvants are substances or procedures that accelerate, extend or enhance the specific immune response against antigens inoculated mucosally or parenterally (Vogel FR. Adjuvants in perspective. In: Brown F, Haaheim LR, editors. Modulation of the inmune response to vaccine antigens. Dev. Bioi. Stand.. Basel. Karger; 1998;92:241-248). Its use generates or potentiates the type of response, and decreases both the number of inoculations and the antigen needed to obtain and maintain protection.

Mucosal adjuvants are those that improve the immune response against antigens administered through the mucosal route. Among the most studied mucosal adjuvants are the enterotoxin of *V. cholerae* (CT), and the heatlabil toxin of *E*. *coli* (LT). The adjuvant activity of CT is related with the ribosilation of ADP (adenosin biphosphate) and the induction of AMPc (cyclic adenosin monophosphate) which has diverse cellular effects (Lycke N, *et al*. 1991. Scand. J. Immunol. 33: 691). The B subunit of CT (CTB) has the ability of increasing the epithelial permeability to heterologous antigens administered nasally, but not to those orally administered (Lycke N, *et al*. 1991. Scand. J. Immunol. 33: 691; Gizurarson S, *et al*. 1991. Vaccine. 9: 825; Gizurarson S, *et al*. 1992. Vaccine. 10:101). It has also been seen that CT induces long-term immunologic memory in intestinal lamina propria of mice (Vajdy M and Lycke NH. 1992. Immunology. 75: 488).

Until now, it has been impossible to selectively separate the adjuvant and toxic effects of CT (Lycke N. *et al*. 1992. Eur. J. lmmunol.; 22: 2277). However, a mutant has been produced with glutaraldehyde as denaturing agent, showing a good retention of adjuvanticity, but 1000 times less toxic (Liang X, *et al*. 1989. J Immunol;143:484).

Some parenterally used adjuvants have also been evaluated through the mucosal route such as immunostimulant complexes (ISCOMs), liposomes, lysophophatidyl glycerol, Avridine (a lipoidal amine) and citokines (Ruby J, et al. 1992. Vaccine. Res.1:347; O'Hagan DT, *et al*. 1992. J Gen Virol;73: 2141).

Immunostimulant complexes have proven to be efficient adjuvants when they are nasally administered. They are relatively stable particles, from 30 to 40 nm, in which most widely used formulation is that containing Quil A (a mixture of saponins extracted from *Quillaja saponaria*) cholesterol and phospholipids in a molar ratio of 1:1:1 (Tomasi M, et al. 1996. Mucosal vaccines. 13:175-186). It has been reported that the ISCOMs modulate the expression of the major histocompatibility complex (MHC) class 11 and could act by stimulating the release of interferon gamma (IFN-γ) (Byars N E and Allison A C. Immunologic Adjuvants: General Properties, Advantages, and Limitations, in Laboratory Method in Immunology, Zola, H., Ed., 39, 1990). It has also been established that they are capable of stimulating CD8+ T cells restricted to MHC class I (Bomford RHR. The differential adjuvant activity of Al(OH)₃ and saponin, in lmmunopharmacology of Infectious Diseases: Vaccine Adjuvants and Modulators of Non-Specific resistance, Madje, J., Ed., Alan R. Liss, New York, 65, 1987). In spite of the advantages of the ISCOMs, in relation to cost, stability and the nature of the antigen that is to be inserted in the membranes, they continue as problems of the immunopotentiation strategy.

Liposomes, the antigen delivery systems, are aqueous suspensions of spheroide vesicles in which the phospholipids it contains are organized in a double layer of lipids. Antigens can be carried into either the vesicles or their surface, according to their hydrophilic or hydrophobic nature, respectively (Childers N K, Michalek S M. Liposomes, in: D.T. Hagan (Ed.), Novel Delivery Systems for Oral Vaccines, CRC Press, Inc., Boca Raton, Florida, 1994). Their adjuvanticity depends on layer number (Susuki M, et al. 1994. Clin Inmunother. 2:121-125), charge (Hadden JW. 1993. Immunology Today. 14: 274), composition (McAnalley BH, *et al.* inventors (Carrigton Laboratories Inc, assignee. Use of acemannan. US patent 229164. 1988 Aug 5; Giles CH, *et al*. J. Soc. Dyers Colour 1958; 74: 647) and production method (Giles CH, *et al*. J. Soc. Dyers Colour 1958; 74: 647, Walkers GJ, 1978. Biochem. Carbohydr. 16:75-126). Their use enhances both humoral and cell-mediated immunity to antigens of protein and polysaccharide nature (Hadden JW. 1993. Immunology Today. 14: 274; Walkers GJ, 1978. Biochem. Carbohydr. 16:75-126, Han YW. 1990. Adv. Appl. Microbial. 35:171-174; Cote G L and Ahegren J A. Metabolism in microorganisms Pert 1. Levan and levansucrase. Science and technology of fructans.1993. pp. 141-168. Edited by M. Susuki &N.J Charton. Boca Raton, FL:CRC Press). On the other hand, the oral administration of an antigen within liposomes produces a higher mucosal response than that obtained by administering the antigen alone through this route (Janeway CA. 1992. lmmunol Today. 5: 3; Paolo C, *et al*. 1999. Vaccine. 17: 12-1263). A great disadvantage in liposomes is that they are destroyed by intestinal lipases and bile salts (Okada J, *et al*. 1995. Pharm. Res.12:576-582).

The use of microencapsulated antigens have also stood out because of its vaccine effectiveness. Microcapsules are spheres with a cover and a core. The cover consist of one or more polymers, whether biodegradable or not, whereas the core consists of the antigen. If the polymer is not biodegradable, then the microcapsule acts as a reservoir with pores through which the antigen escapes slowly. If the polymer is biodegradable, the antigen is released through the degradation of the microsphere. The latter is more frequent case and an example of this are frequently used encapsulated microspheres with the co-polymer of lactic acid and glycolic acid. Microspheres have been used nasally (Eyles JE, *et al*. 1999. Int J Pharm. 189(1):75-9), oral and parenterally (Gupta R K, *et al*. 1997. Vaccine; 15(16):1716-1723).

Virus-like particles (VLP) have also been used in antigen delivery systems for strategies of mucosal immunization. VLP consist of viral capsides and envelopes, or other proteins that when assembled in supra-molecular structures they resemble viruses. They have the advantages of simple production and purification, and as particulated antigens they are better than soluble antigents for the induction of mucosal immune responses (André FE. 1990. Vaccine; 8 (S74)). The nasal administration of human papilloma virus particles (HPV VLP) has shown good results in generating humoral and cellular responses (Dupuy C, *et al*. 1999. Journal of virology; 73:11:9063-9071; Liu XS, *et al*. 1999. Virology. 252:39-45, Balmelli C, *et al*. 1998. J of Virol. 72:8220-8229).

Although there are several inductor sites of the mucosal immune response the most convenient one is NALT.

Vaccination through the intranasal route with live influenza vaccines has given good results in children and adults. This route can be useful for other vaccines sensitive to the gastrointestinal conditions when given orally (Walker R I. 1994. Vaccine. 4:387).

In 1997 the first study in humans showed that vaccination vaccination through the intranasal route with the recombinant cholera toxin B subunit (rCTB) induces specific IgA and IgG in vaginal secretions (Bergquist, Ch. 1997. Infection and immunity. 65, 2676). Furthermore, animal immunization by this route has generated an IgA response in vaginal secretions that is even higher than the immunization through the intravaginal route (Di Tommaso A. 1996. infect. Immun.64: 974; Gallichan W S and Rosenthal K L. 1995. Vaccine. 5:1589; Hopkins S, 1995. Infect. Immun. 63:3279).

All antigens used in this invention have in common, among other things, that they have been widely investigated with vaccine purposes, even many of them by the nasal route. However, although there seem to be reported in a large number of studies, these have mainly used the parenteral route and they have never been used through the intranasal route with HBsAg.

The nasal administration of the hepatitis B surface antigen (HBsAg) (1 or 5 µg) together with the recombinant cholera toxin B subunit (10 µg) also given nasally to mice generates not only systemic immune responses against HBsAg, but mucosal responses are also found in nasal cavities, lungs, saliva, the small intestine and the vagina. High levels of serum IgG1 specific to viral antigen, IgG2a and IgG2b were obtained with this combination. Sera titers in almost all mice, measured by EIA sandwich using a commercial kit, were higher than 1000 mlU/ml) (lsaka M. *et al*. 2001. Vaccine. 19(11-12):1460-1466).

It has been demonstrated that the nasal immunization of BALB/c mice with HBsAg and oligodeoxynucleotide with CPG motifs (CpG ODN) produce an antibody immune response against the viral antigen of the same magnitude as that produced by CT or LT with HBsAg, and higher than that of the combination of CTB or LTK63 (a mutant of LT) with HBsAg. Furthermore, the simultaneous use through the intranasal route of CpG ODN, CT (or LT) and HBsAg produce a synergistic effect on the immune response against this last antigen, but not when it is used with CTB or LTK63 instead of toxins (McCluskie MJ, *et al*. 2000. Mol Med Oct; 6(10):867-877). The predominating isotypes after the administration of CpG ODN and HBsAg are IgG1/IgG2a, while the additional administration of CT mainly IgG2a is produced (McCluskie MJ, *et al*. 1998. J lmmunol. Nov 1; 161(9):4463-4466).

It has been found that the administration of acemannan (an acetyled polymer of mannose extracted from the plant *Aloe barbadensis* Miller) together with HBsAg by the intranasal route generates serum IgG antibody response similar to that obtained with the administration of the antigen adjuvanted in alum, as well as an IgA response in vaginal secretions, comparable to that obtained by the nasal application of HBsAg adjuvanted with the cholera toxin (Aguilar JC *et al*. 1998. WO 9839032).

The strong adjuvant effect exerted by the hepatitis B virus core antigen (HBcAg) on HBsAg when they are both inoculated through the intranasal route has also been verified. These effect have proven to be of a similar magnitude to that of CT under equal conditions, and even higher than that shown by alum (administered intramuscularly) in the induction of serum IgG antibody response (Aguilar JC, *et al*. PCT/CU/99/00006). This reference, a patent application of our team, showed the presence of a synergistic effect in the cross immunoenhancement between different VLP when they are administered mucosally. VLP combinations of HPV, HCV and HBV respectively were used in this study, reaching any possible combination of VLP in which HBsAg be included. Thus, a potential cross enhancing activity is already evidenced between different VLP (HBsAg is a VLP). It is not obvious that other non-VLP antigens receive any enhancer effect due to their interaction with HBsAg. There are not described possible formulations of HBsAg with and other antigens than VLP (Aguilar JC, *et al*. PCT/CU/99/00006).

Diphtheria toxoid (DT) covers an important amount of the current vaccine literature, fundamentally by parenteral routes. There are some papers where it has been employed by the nasal route. The parenteral priming with diphtheria toxoid in alum, followed by an nasal booster with CRM₁₉₇ is an immunization method that very effective in mice, capable of inducing high levels of anti- DT IgG and neutralizing antibodies in sera and secretory IgA in the respiratory tract (McNeela EA, et al. 2000. Vaccine. Dec 8;19(9-10):1188-98).

When the recombinant heat- labile enterotoxin B subunit of *E*. *coli* (rLTB) was administered nasally to mice together with DT, it produced a substantial stimulation of serum DT-specific IgG antibodies and a moderate induction of mucosal DT-specific IgA in the nasal cavities and lungs (Kozuka S, *et al*. 2000. Vaccine. Mar 6;18(17):1730-7).

Nasal immunization of 5 Lf of DT together with the recombinant cholera toxin B subunit (CTB) induced high serum DT-specific IgG antibody responses or moderate specific IgA responses in all mice and just slight IgE antibody responses in some mice. Moreover, there were sufficiently high titers of diphtheria antitoxin, more than 0.1 IU/ml, in mice showing high levels of serum DT-specific IgG antibody responses. Under the same experimental conditions, the induction of significant mucosal DT-specific IgA antibody responses occurred in nasal cavities, lungs, saliva, vaginal secretions and small and large intestines of all mice (lsaka M, *et al*. 1999. Vaccine. Nov 12;18(7-8):743-51).

The use of a new suspension containing mono-olein/oleic acid vesicles together with DT, administered parenteral or nasally to mice has been reported to increase the toxoid immunogenicity to the same level as alum adsorbed or administered in Freund's complete adjuvant. This study shows a relationship between immunogenicity and acyl chain length (Schroder U, *et al*. 1999 Vaccine. Apr 9;17(15-16):2096-103).

Tetanus toxoid, like DT, is also noted for covering important part of current vaccine literature, mainly in parenteral immunization strategies.

With the aim of evaluating the induction of mucosal IgA antibody responses using interleukins 6 and 12 (IL-6, IL-12) together with tetanus toxoid (TT) administered nasally, a study was carried out that showed that the simultaneous administration of IL-6 with TT to mice induced serum TT-specific IgG antibody responses (mainly IgG1 and IgG2b) higher than in the control mice, but low secretory IgA antibody responses and no IgE. In contrast, IL-12 administered nasally together with TT, not only induced a sharp increase of serum IgG, but also enhanced IgA antibody response in the mucosa. The co-administration of IL-6, IL-12 and TT did not increase serum or mucosal antibody responses compared to those produced by the combination of IL-12 and TT (Boyaka PN, *et al*. 1999. J lmmunol. Jan 1;162(1):122-8).

The nasal administration of IL-12 to mice that had been nasally immunized with TT and CT adjuvant resulted in an increase of TT-specific IgG2a and IgG3 antibody levels while IgG1 and IgE antibody responses decreased markedly. In contrast nasal IL-12 enhanced CT-induced serum IgG1 and IgE antibody responses in mice given a mixture of TT and CT orally (Marinaro M. et al. 1999. J lmmunol. Jan 1;162(1):114-21).

In nasal immunization experiments with TT formulations the systemic and mucosal responses of mice immunized with TT adsorbed in alum and mixed with rCTB were examined in the case of the nasal administration of non-adsorbed TT 5 Lf were necessary to stimulate, only in the presence of rCTB (10µg), high serum TT-specific IgG in all mice examined, and moderate or slight TT-specific IgA antibody responses in nasal, lung and intestinal lavages of a few mice, showing that its immunogenicity through the nasal root is poor. Nevertheless, after it is reached, it may resist the challenge with tetanus toxin (Isaka M, *et al*. 1998 Vaccine. Oct;16(17):1620-6).

TT has also been used as a model in testing new adjuvants like the nontoxic mutant CT: CTS61 F. A comparative study on immune response generated by the nasal administration of this protein with several antigens separately (TT among them) and of those obtained following a similar protocol with native CT and a rCTB. Serum TT-specific IgG, IgA and IgM responses, as well as IgA antibody response in mucosal secretions increased significantly in both the formulation containing native CT and that containing the mutant CT; rCTB did not show a good adjuvant activity (Yamamoto S. *et al*. 1997. Proc Natl Acad Sci U S A. May 13;94(10):5267-72).

Nasal administrations to guinea-pigs of tetanus toxoid adsorbed onto poly (L-lactic acid) microspheres enhanced the immune response with respect to that obtained with free antigen; the latter was similar to that found in non-immunized animals (Almeida AJ, *et al*. 1993. J Pharm Pharmacol. Mar;45(3):198-203).

The determination of immunologic responses, particularly the immunopathological reactions associated with the nasal administration of the mucosal adjuvant CT was the aim of a study in which TT and CT were combined and administered to BALB/c mice. After nasal immunization, mice produced an antibody response in serum, mainly of the IgG isotype, predominantly the IgG1 subclass, against both TT and CT. Together with antibody response there were also inflammatory reactions in lungs that could be potentially fatal. Furthermore, there were induced IgE responses, which were associated with interleukin 5 (IL-5) detection in sera. Thus it was suggested that nasal immunization with TT plus CT would likely result in the activation of Th2 cells, which may contribute to serious immunopathologic reactions in the lungs (Simecka JW, *et al*. 2000. Infect Immun. Feb;68(2):672-9). This highlights the importance of a rational design of immunization strategies producing a savings in resources, such as the search for strategies that substitute the toxic adjuvants used as a model in studying the immunogenicity and efficacy of the different routes, but that have combinations that are not applicable to humans.

Another antigen universally utilized in human vaccines is formaldehyde-inactivated *Bordetella pertussis* (Bp). This bacterin, administered by the intranasal route to BALB/c mice induces high levels of IgG antibodies in the serum and bronchoalveolar fluids, as well as IgA in the serum and broncoalveolar fluids, saliva, and faeces. However, when it is administered together with CT, anti-Bp IgG responses are not enhanced whereas IgA responses significantly decrease in all secretions analyzed (Berstad AK, *et al*. 1997. Vaccine. Aug-Sep;15(12-13):1473-8).

To test for the nasal immunogenicity and adjuvant ability of Bp a study was carried out in mice in which this antigen was nasally administered together with the inactivated influenza virus. The virus alone induced low levels of influenza-specific serum IgG antibodies, though they were significantly higher than the non-immunized controls, whereas there were no differences between serum- and saliva- IgA responses. In contrast, when Bp was administered together with the inactivated influenza virus, serum virus-specific IgA and IgG and salivary IgA responses were substantially enhanced (P<0.005). However, this adjuvant effect was not significant for the same type of response in the gut (measured as antibodies in faeces). On the other hand antibody responses against Bp were inhibited by mixing with the viral vaccine. Saliva antibodies generated against Bp showed cross-reactiveness with *Neisseria meningitidis* (Berstad AK *et al*. 2000. Vaccine. Mar 17;18(18): 1910-9). This is important because it demonstrates that it is not obvious that starting from an antigen combination, a higher response is induced for all the antigens present in the combination. Bp has also been tested in humans. Six adults were administered cellular pertussis vaccines four times through the nasal route, at weekly intervals. All vaccinees responded with increases in nasal fluid IgA antibodies to Bp whole-cell antigens. Three vaccinees with high nasal antibody responses also developed increased serum IgA and IgG antibody titers against Bp. Salivary antibody responses to the whole-cell antigen, as well as antibodies in serum and secretions to pertussis toxin (PT) and filamentous haemagglutinin (FHA) were negligible except for a moderate increase in nasal fluid antibodies to FHA. Unexpectedly, in the same vaccinees there were significant rises in nasal and salivary antibodies to meningococcal outer-membrane antigens, whereas corresponding serum IgA and IgG antibodies were unchanged (Berstad AK, *et al*. 2000, J Med Microbiol. Feb;49(2): 157-63). That is why a nasal formulation against this antigen should take into account the response against individual proteins in the immunogenicity study.

Although the presence of serum bactericidal antibodies has been correlated with an immunity to meningococcal diseases, mucosal immunity at the port of entry may also play an important role. That is why a study was carried out to evaluate the immunogenicity of a *Neisseria meningitidis B* outer-membrane complex (OMPC) in an nasal vaccine formulation given to mice. In this study a strong systemic bactericidal antibody response as well as a strong local IgA response in lungs was evidenced. However, 8- to 10-fold-higher doses of OMPC were required in nasal immunizations compared to intra-peritoneal immunizations to elicit an equivalent bactericidal antibody response in serum (Saunders NB, *et al*. 1999. Infect lmmun Jan;67(1): 113-9).

The ability of the Norwegian group B meningococcal outer membrane vesicle vaccine to induce a T-cell response in humans has been verified, after its nasal administration without an adjuvant. To achieved this, a group of 12 individuals were immunized with four doses of OMPC (250 µg of protein/dose) at weekly intervals, and a booster dose 5 months later. T-cell proliferation in response to the OMPC vaccine, purified PorA (class 1) protein, PorB (class 3) protein, and one unrelated control antigen (*Mycobacterium bovis* BCG) was measured by [3H]thymidine incorporation into peripheral blood mononuclear cells obtained before and after the immunizations. Nasal immunizations with OMPC induced antigen-specific T-cell responses in the majority of the vaccinees when tested against OMPC (7 out of 12) and the PorA antigen (11 of 12); none of the vaccinees showed a vaccine-induced T-cell response to the PorB antigen after the initial four doses (Oftung F, *et al*. 1999. Infect Immun. Feb;67(2): 921-7).

It has been demonstrated in humans that OMPC administered in the form of nose drops or a nasal spray four times at weekly intervals leads to the development of nasal and salivary IgA responses. Moreover, modest increases of serum IgG antibodies have been observed in several immunized individuals (Haneberg B, *et al*. 1998. infect Immun Apr;66(4): 1334-41).

On the other hand, the additional use of CT in mice by mucosal routes (nasal and rectal) enhances serum antibody responses compared to the OMPC vaccine administered by the same routes. However, the most effective immunizations have been the nasal ones so it is deducted that mucosal responses are not dependent on the use of CT. Besides, the serum bactericidal activity is similarly not enhanced by CT, indicating that the positive effect on the serum IgG level does not include bactericidal activity (Dalseg R, et al. 1999. Vaccine, May 14;17(19): 2336-45).

It has been seen that the use of OMPC in a complex with the lipopolysaccharide (LPS) of *Brucella melitensis* generates, by nasal administration to mice, high levels of anti-LPS IgG and IgA in lung mucosa, as well as IgG and IgA antibody-secreting cells in the lungs and spleen after the inoculation of two doses. On the other hand, high levels of serum IgG and moderate levels of IgA are also found in the serum. It has been suggested, due to the prominent IgG1 subclass response obtained, that OMPC may favor a Th2-like response to the LPS (Van De Verg LL, *et al*. 1996. Infect Immun Dec;64(12):5263-8).

The possibility of having vaccines containing several antigens derived from different pathogens has been fundamental in the development of the Expanded Immunization Program promoted by the World Health Organization, and here, there is an attempt to include the hepatitis B vaccine (Chiu HH, *et al*. 1998. Pediatr Infect Dis J Mar;17(3):206-11).

It has been demonstrated that the administration of a vaccine containing HBsAg, DT, TT and Bp (5 to 10 µg of HBsAg) to healthy children at 1.5, 3.5 and 6 months of age, if they were immunized at birth with a HBsAg vaccine (10µg), produces protective serum anti- HBsAg antibody titers (more than 10mlU/ml) (Chiu HH, *et al*. 1998. Pediatr Infect Dis J Mar;17(3):206-11).

Antibody responses against the HBsAg, DT, TT and Bp antigens is not affected by the parenteral administration of a vaccine containing the capsular *Haemophilus influenzae-*type b polysaccharide (PRP) conjugated to tetanus toxoid (PRP-TT), HBsAg, DT, TT and Bp antigens to infants or when two vaccines are administered by the same route, one with HBsAg, DT, TT and Bp antigens, and the other with only PRP-TT. Antibody response against the first four antigens is not affected by an application of a formulation obtained from the mixture of the vaccine containing HBsAg, DT, TT and Bp antigens used to reconstitute lyophilized PRP-TT. On the other hand, the anti- PRP antibody response is significantly lower in the latter case (Greenberg DP, *et al*. 2000. Pediatr Infect Dis J. Dec;19(12):1135-40).

Vaccination with a formulation containing only HBsAg and PRP (the latter conjugated to OMPC of *N*. *meningitidis*) to healthy adults who had previously been exposed to these antigens, increases serum antibody levels against the antigens (Bulkow LR, et al. 1993. Arctic Med Res Jul;52(3): 118-26).

The addition of PRP to a vaccine containing HBsAg, DT, TT, Bp antigens and inactivated polio virus does not produce either a decrease in the immunogenicity of the second antigens or an increase in reactogenicity in humans. On the other hand, the anti- PRP antibody titers produced with the new formulation are similar to those obtained with PRP-monovalent vaccines, or combinations of PRP with DT, TT and Bp antigens that are licensed in certain European countries (Schmitt HJ, *et al*. 2000. J Pediatr Sep;137(3):304-12).

Currently, there is no reference on studies of antigenic combinations related to the nasal administration of HBsAg and DT, TT, OMPC, Bp, Hib or other soluble antigens or resulting from a viral or bacterial inactivation evidencing the enhancing effect of HBsAg. Among the administration advantages of a combined vaccine through the intranasal route is the possibility of reducing the number of administrations, bearing in mind that there will be more antigens at one time and not each one separately; also, it is possible to not include adjuvants, based on the properties of some antigens to increase the immunogenicity of others without considerably affecting negatively its own, the possibility of doing without specialized personnel and medical materials, which complicates vaccine application and makes it more expensive; the fact that no invasive method is used, increases the quality of life of the persons to be immunized, mainly children; and, it is possible to obtain the same or a better protection than that achieved through parenteral vaccines, even in critical ages as childhood and senility, because of the generation of responses at mucosal levels, the main port of entry of many pathogens.

### Detailed description of the invention

The present invention is related to the use of the hepatitis B virus surface antigen as an immunoenhancer in nasal immunizations, with vaccine formulations resulting from the combination of this antigen and other vaccine antigens that benefit from this property and the application of this property of the hepatitis B virus surface antigen and the use of formulations in the field of vaccines.

It is also related with multivalent formulations, specifically for nasal administration resulting from the application of this property of the HBsAg, favoring an increase in the immune response of other antigens present in the formulations.

Vaccine formulations for the nasal administration of this invention together with the hepatitis B virus surface antigen may contain one or more protein antigens of a soluble nature, receiving an immunoenhancing effect due to its co-administration with HBsAg. These may be: tetanus toxoid, diphtheria toxoid or protein-polysaccharide conjugates where the saccharide part corresponds to a vaccine candidate anti *Haemophilus influenzae* type b, the C polysaccharide of *Neisseria meningitidis,* the vaccine polysaccharide of *Pneumococcus pneumoniae,* or in general, one or more soluble proteins of vaccines of interest either purified or obtained recombinantly.

Also within the aim of this invention are the multivalent formulations for nasal administration in which the hepatitis B virus surface antigen is combined with a vaccine candidate from inactivated microorganisms which receives an immunoenhancing effect because of its co-administration with HBsAg. The vaccine antigen may be *Bordetella pertussis* whole-cell, which receives an immunoenhancing effect because of its co-administration with HBsAg or other vaccine antigens of the same nature alone, or integrating complex combinations of antigens.

Other vaccine antigens that may be contained are the present inactivated or attenuated vaccine candidates.

Vaccine formulations for nasal administrations related with the present invention may contain an n number of antigens from microorganisms of different species ranging from n=1 to n=20, of antigenic nature among those previously described, with a final volume and antigen amounts for inoculation ranging from 50 microliters to 2 milliliters, and from 0.1 micrograms to 2 mg, respectively, depending on the size and the species to be immunized.

Formulations of the present invention may be solubilized in PBS, saline solution, water for injection or in any buffer solution used in medical practice that allows for antigen stability, in an antigen concentration lying within the possible combinations of mass and volume previously described.

Likewise, the antigenic components can be mixed with HBsAg according to the candidates of interest in relation to vaccination age or to multivalent candidates based on any other premise, where one, two or more antigen types described above are presented either lyophilized or administered in drops, sprays or pulverization.

Vaccine formulations of the present invention can be used to attain an effective immunization in humans or animals as a preventive or therapeutic treatment.

### Brief description of figures

Figure 1. Kinetics of IgG response of an experiment where inoculations were achieved on days 0, 14, 28 y 87 and bleedings on days -10, 21, 35, 42, 84 and 97. (A) Kinetics of serum IgG response against HBsAg; (B) Kinetics of serum IgG response against TT; (C) Kinetics of serum IgG response against TD and (D) Kinetics of serum IgG response against Bp. Tables 1A, B, C and D: results of the statistical analyses of the comparisons between groups of the corresponding figures.
Figure 2. Vaginal IgA response on day 97. (A) IgA response against HBsAg; (B) IgA response against TT; (C) IgA response against DT and (D) IgA response against whole-cell Bp. Tables 3A, B, C and D: statistical analyses.
Figure 3. Lung IgA response on day 97. (A) IgA response against HBsAg; (B) IgA response against TT; (C) IgA response against DT and (D) IgA response against whole-cell Bp. Tables 3A, B, C and D: statistical analyses.
Figure 4. Evaluation of serum IgG response against individual proteins of Bp, after the administration of tetravalent formulations by the intra-peritoneal or nasal routes. Optical density levels generated by individual sera of each group immunized with nasal or intra-peritoneal tetravalent formulations (groups 7 and 13, respectively), after (A) a third inoculation and (B) a fourth inoculation.
Figure 5. Evaluation of the proliferative activity against antigens found in the nasal tetravalent formulation of the example 1, administered individually and within the tetravalent formulation.
Figure 6. Enhancing activity of HBsAg on *Neisseria meningitidis* OMPC. The combination of both antigens significantly increased the anti- OMPC response.

### EXAMPLES

### Example 1

With the aim of evaluating the antibody response generated after the nasal administration of several formulations containing different types of antigens together with or without HBsAg, an experiment was designed with 126 female BALB/c mice, 8 to 10 weeks of age, divided into 13 groups: groups 1 to 11 with 10 animals each, and groups 12 and 13 with 8 animals each. All mice were immunized at days 0, 14, 28 and 87 and bled at days -10, 21, 35, 42, 84 and 97.

The dose of each antigen administered per mouse is shown below:

| **Group** | **Route** | **Dose of antigen per group** |
|---|---|---|
| Group 1 | (nasal)¹ | 5µg of HBsAg + 10µg of DT |
| Group 2 | (nasal) | 5µg of HBsAg + 3.2 UOP of Bp* |
| Group 3 | (nasal) | 5µg of HBsAg + 10µg of TT |
| Group 4 | (nasal) | 5µg of HBsAg + 10µg of DT + 3.2 UOP of Bp* |
| Group 5 | (nasal) | 5µg of HBsAg + 10µg of DT + 10µg of TT |
| Group 6 | (nasal) | 5µg of HBsAg + 3.2 UOP of Bp*+ 10µg of TT |
| Group 7 | (nasal) | 5µg of HBsAg + 10µg of DT + 10µg of TT + 3.2 UOP of Bp* |
| Group 8 | (nasal) | 5µg of HBsAg |
| Group 9 | (nasal) | 10µg of DT |
| Group 10 | (nasal) | 3.2 UOP of Bp* |
| Group 11 | (nasal) | 10µg of TT |
| Group 12 | (IP)² | 5µg of HBsAg + 0.125 mg of AI(OH)₃ |
| Group 13^{ψ} | (IP) | 5µg of HBsAg + 49.26 µg of DT + 29.07 µg of TT + 8.0 UOP of BP* + 0.125 mg of Al(OH₃) |

| | | |
|---|---|---|
| (nasal)¹ Group with nasal immunization | | |
| (IP)² Group with intra-peritoneal immunization | | |
| * UOP: Units of Opacity; in each case equal amounts of UOP of both Bp strains described above, were used. | | |
| ^{ψ} It can be observed that group 13 had the same HBsAg dose **as the** nasal groups. However, the doses of the remaining antigens here were higher because a commercial vaccine formulation was used as the control. This vaccine contained specific doses of each antigen that could not be changed. These correspond to the micrograms presented in the table. Hence the amount of TT, DT and Bp used through the intranasal route are 3, 5 and 2.5 **fold** less respectively. The same amounts of HBsAg were nonetheless used. | | |

### Serum anti- HBsAg antibody response

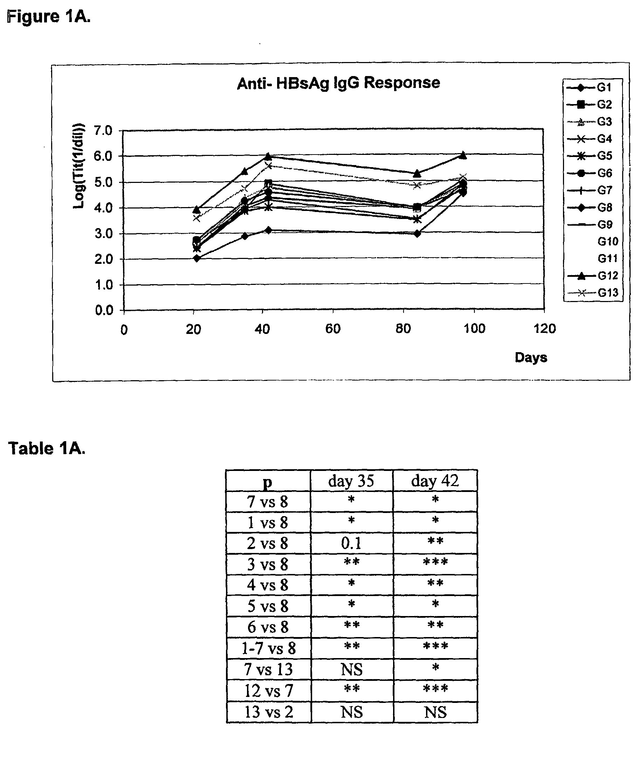

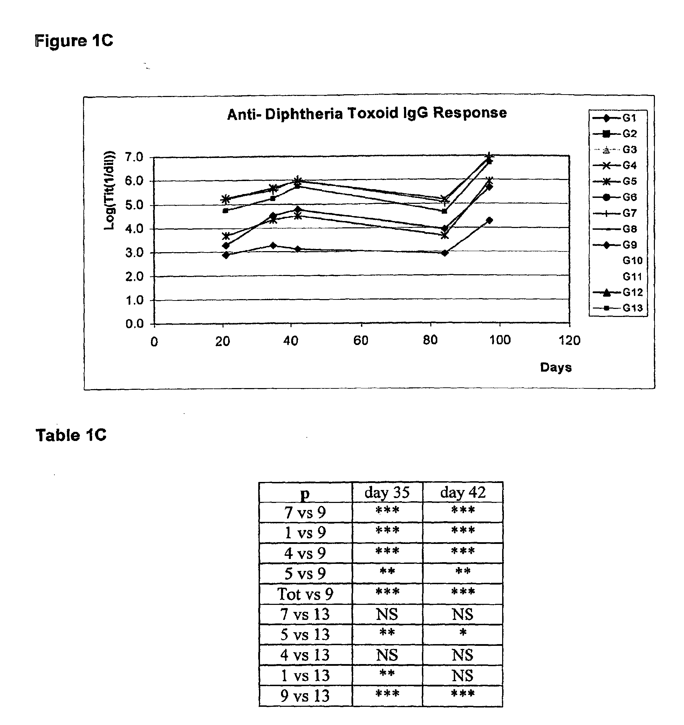

Determinations of IgG antibody response against the hepatitis B virus surface antigen indicated that a week after the third administration, in the group nasally immunized with HBsAg, DT, TT and Bp (group 7) it was significantly higher than group 8, which only received HBsAg in PBS by the same route. This behavior was repeated on day 42 (Figure 1A).

The other nasally immunized groups (groups 1-6) showed the same behavior as group 7, developing IgG levels that are significantly greater than those obtained with HBsAg in PBS after three doses, except for group 2, which had a higher, but not significant (p>0.05) response on day 35. Nevertheless, this group did have a very significant difference on day 42, which can be explained by a broad dispersion of the titers on day 35.

The groups nasally immunized with formulations containing HBsAg and Bp; HBsAg, Bp and DT; and HBsAg, Bp and TT (groups 2, 4 y 6 respectively) had significantly higher IgG responses than those shown by group 8 after the second dose.

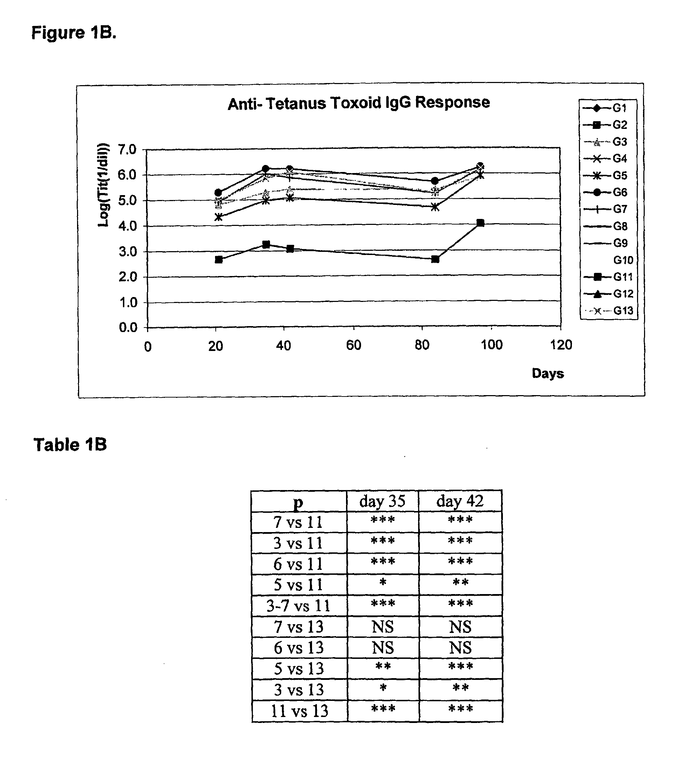

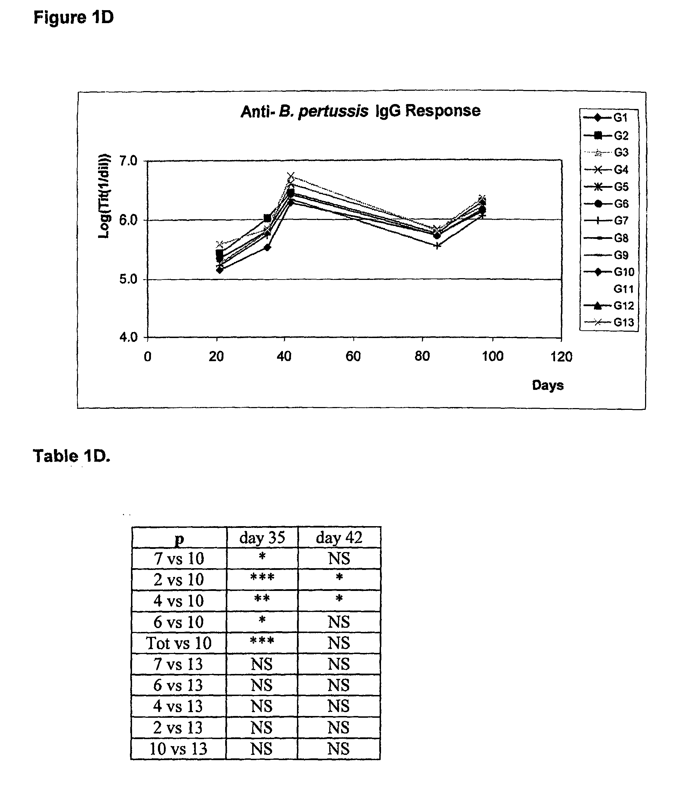

It is good to point out that on day 35, the anti-HBsAg IgG response of the group that was nasally immunized with the HB-DTP mixture was not significantly different from that of the homologous group immunized through an intra-peritoneal injection (group 13). In the same way, after four doses, statistically similar values were obtained between nasal immunized groups and the IP immunized group using the antigen combination (Fig. 1).

Anti-HBsAg titers generated in the group immunized with the Heberbiovac HB® vaccine (group 12) were significantly higher than those obtained with any nasal group. This result is due to a higher immunogenicity of HBsAg when it is administered by the intra-peritoneal (IP) route with respect to intramuscular and subcutaneous routes. This characteristic of the IP route has been shown in our experiments. In spite of this characteristic of the anti-HBsAg response, the inoculation of 250 µl of the vaccine per mouse, corresponding to the dose of 5 µg of HBsAg -equivalent to nasal dose- makes it necessary to use of IP route. Nevertheless, it has been observed in older animals that the response generated by the nasal inoculation equals that induced by intra-peritoneal injection. An example of this was seen in the second experiment (see example 2) though the nasal candidates were different.

Despite a lower titer intensity generated by the nasal route, it has been recently observed that anti-lgG2a response levels induced in group 2 were significantly higher than those shown by group 12 (data not shown). This demonstrates a modulating effect of Bp on the anti-HBsAg antibody response, introducing a qualitative change in the response, favoring the Th1-like response, which is evidenced by the IgG subclass profile. This response is characterized by a higher production of IgG2a antibodies with respect to the common profile generated by the vaccine adjuvanted in alum.

As observed in Fig 1, the appearance and sustainment of the nasal and intra-peritoneal anti- HBsAg responses were very similar, with a similar increase and decrease in time.

### Serum anti- tetanus toxoid IgG antibody response

A stronger immunoenhancing effect of HBsAg, DT and Bp on tetanus toxoid was evidenced after their nasal administration in the group 7mixture. After the second, third and fourth inoculations, the anti- TT IgG levels of this group showed a highly significant increase with respect to group 11, immunized with tetanus toxoid in PBS by the same route.

The other groups nasally vaccinated with mixtures containing TT, specifically groups 3, 5 and 6, showed higher antibody levels to those induced in group 11 after each bleeding. In the case of group 3, an antibody level approximately 100 times higher than group 11 was attained after the second dose. This is highly relevant because this is the first demonstration of the immunoenhancing activity of HBsAg on a soluble antigen such as TT.

As observed in the anti- HBsAg response, tetanus toxoid also increased the anti-HBsAg response, showing that we are in the presence of a synergistic interaction with respect to a cross- enhancement of immunogenicity. This type of phenomenon had already been observed for HBsAg and HBcAg and other viral nucleocapside antigens, but never with a soluble antigen. TT is not immunogenic by the nasal route, which is evidenced in group 11, immunized with the toxoid in PBS. Hence, this effect was not expected with HBsAg and TT.

Anti-TT response was strengthened significantly more when HBsAg, TT and Bp were formulated together (group 6) producing anti- TT responses 1000 times stronger than those generated by intranasal immunization with TT in PBS (group 11). This additional increase was not obtained by the mixture of DT, HBsAg and TT (group 5). This formulation did not produce a significant change, but showed a statistical behavior similar to that of the combination of TT and HBsAg in respect to anti- TT response (Fig. 1B).

Titers of groups 6 and 7 were not significantly different. Both these groups having in common the presence of Bp and HBsAg, induced responses that were 1000 times higher. Therefore, we can say that both the surface antigen and Bp produce an immunoenhancement of the anti-TT response that increases titers to very significant levels in the first case and to highly significant levels with the mixture of both. Since all groups of intranasal combinations in the experiment were immunized with HBsAg, it is important to define whether the enhancing effect of Bp is independent of HBsAg or whether both have a synergistic enhancing activity. It can be affirmed with respect to TT, that the number of antigens present in all formulations studied did not affect its immunogenicity, but it was increased still more, reaching highly significant levels (Fig. 1B).

The TT-specific IgG levels induced in groups 7 and 13, both immunized with the 4 antigens by the intranasal and intra-peritoneal administration respectively, did not show significant differences. Responses were, however, obtained through the intranasal route for certain groups that were higher, although not significantly so, compared to those obtained in group 13 (Fig. 1 B).

Although the groups immunized with HBsAg and TT (group 3) and with HBsAg, DT and TT (group 5) did not have different IgG titers to those obtained in group 13, a week after the second dose, they had a significantly lower response after the third and fourth doses. This result evidenced that it is important to add Bp to the mixture in order to increase anti-TT response to levels similar to those obtained after IP administration. Nevertheless, it should also be taken into account that amounts of TT 3 times higher were administered by the IP than by the nasal route.

### Response of IgG antibodies anti- diphtheria toxoid in serum

The nasal administration of DT together with HBsAg, TT and Bp (group 7) led to a considerable increase in DT immunogenicity, compared to the response reached by group 9, in which DT was administered in PBS (Fig. 1C).

The same behavior was shown in the rest of the groups nasally immunized with DT combinations (groups 1, 4 and 5) in highly significant increases in anti-DT IgG levels. Only group 1 did not show a significant superior result after second dose. However, 35 and 42 days after the third dose, the response of group 1 was highly significant compared to the group immunized with diphtheria toxoid in PBS (Fig. 1C).

The comparison of anti-DT IgG levels between nasally immunized groups with formulations containing DT and the group with the intra-peritoneal administration, the tetravalent commercial vaccine (group 13) showed an anti-TT response-like behavior. In other words, the response was enhanced 100 fold in groups containing HBsAg but not Bp, whereas the addition of Bp allowed to increase the responses still more, even to levels higher than those generated by the IP route with the DPT-HB vaccine. This behavior of DT reproduces the effect found for the other soluble protein, TT. Hence, we can affirm that HBsAg has a strong immunoenhancing effect on both soluble proteins and vice versa. In the case of DT, a synergistic activity was also evidenced with regard to the cross-enhancement of the immune response to the antigens into the mixtures; hence, as it was explained before, anti- surface antigen IgG response was enhanced by the addition of DT. Once more the need of adding Bp enabling to increase the anti-DT response one-hundred times, to levels similar to those obtained after IP administration, was demonstrated. It must be recalled that the response is similar to that obtained through the IP route although in that case the amount of DT was five times larger.

### Anti- B pertussis whole cell IgG antibody response in the serum

All anti- Bp responses were characterized by their strength and quick increase to levels near saturation (Fig. 1D). In spite of the resulting homogeneity, statistical analysis revealed the possibility of the generation of significant differences between groups.

After a third administration the group nasally immunized with the tetravalent combination generated a significantly higher response than that obtained in the group immunized with Bp alone through the same route (group 10). However, this effect disappeared after the fourth inoculation since it rapidly reached a state of titer saturation.

The other groups nasally immunized with formulations containing Bp together with one or more antigens had strong responses, that were significantly to highly significant greater in the case of the group given HBsAg and Bp on day 35, evidencing that the immunoenhancing effect of HBsAg is also produced in inactivated cells, in addition to the known effect on soluble proteins. Both groups 2 and 4 maintained this statistical difference on day 42, while groups 6 and 7 (having administered the more complex combinations) did not differ from group 10.

A comparison of all intranasal groups with the IP group showed no significant differences after the second and third doses, evidencing that high titers can be obtained by nasal immunization with the whole cell.

### Example 2.

### Determination of mucosal response of the nasal multivalent formulations of HBsAg.

Taking into account that a stronger response to the nasally administered antigens depends on whether a strong response can be generated at the mucosal and systemic levels, we determined IgA antibody response in vaginal and lung lavages in the immunized groups that are described in example 1.

### Vaginal anti- HBsAg response

After the fourth administration, on day 100, HBsAg-specific IgA response induced in vaginal lavages of the nasally immunized group with the tetravalent formulation, was not significantly different from that found in mice exclusively immunized with HBsAg through the same route. It is important to point out that at this time, serum anti-HBsAg IgG titers were not different either because of the strong response generated in the group immunized with HBsAg in PBS by boosting. However, a 20% higher seroconversion was found in the first group. Although no differences were found, it should be highlighed that such a large amount of antigens does not affect vaginal anti- HBsAg response.

On the other hand, when comparing IgA levels induced in the groups receiving the combination of the four antigens either through the intranasal or intra-peritoneal routes (groups 7 and 13 respectively) it was demonstrated that the levels of predominant antibodies in vaginal secretions, developed in the group immunized through the mucosal route were significantly higher than those of the parenterally immunized group. It should be stressed that the intra-peritoneal inoculation with HBsAg formulations results in a vaginal IgA level, that although low, its value is higher than those obtained by the SC and IM routes (data not shown). That is why the response found in vaginal lavages after IP immunization were significant. In general, intranasal immunization exceeded IP, as expected.

### Vaginal anti- tetanus toxoid response

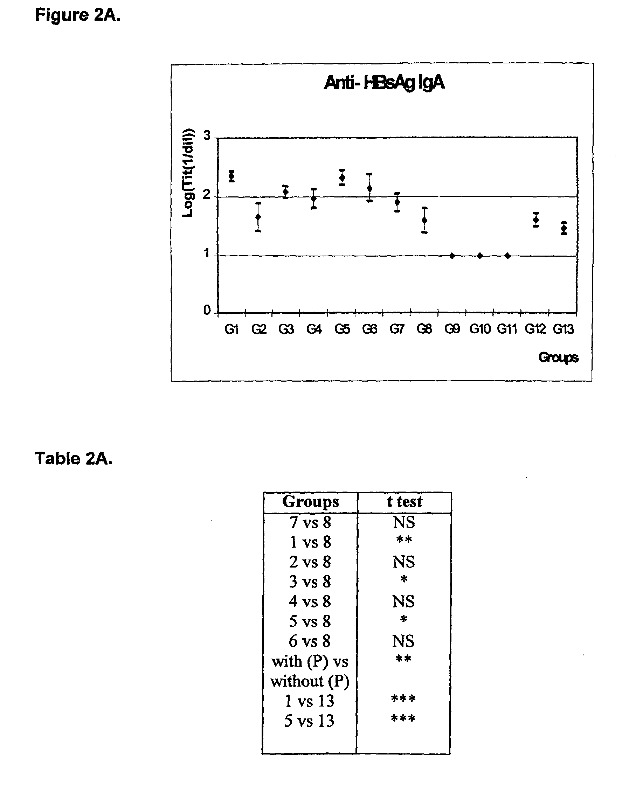

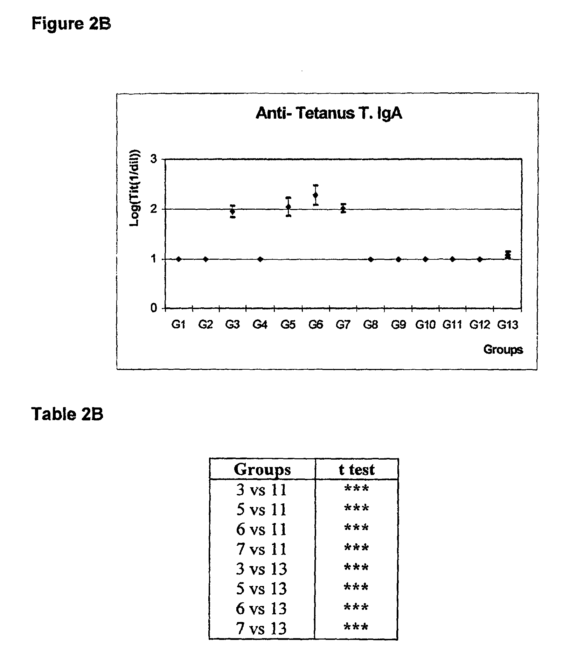

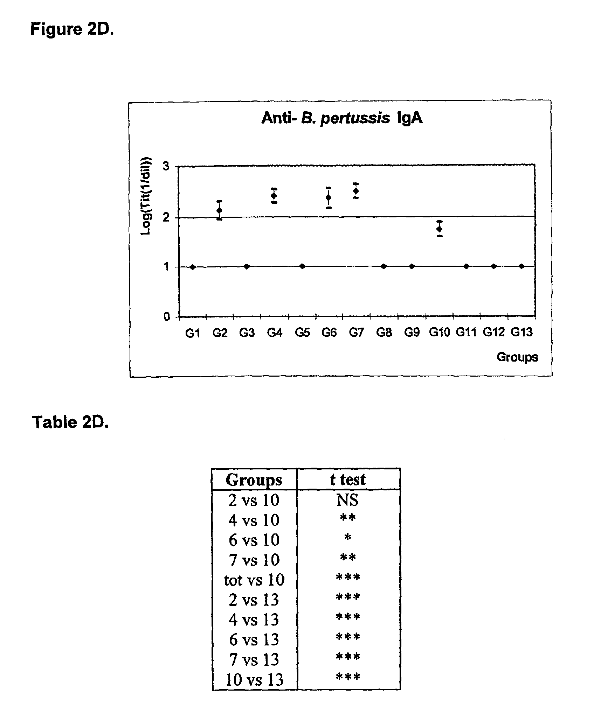

The determination of vaginal TT-specific IgA levels after four administrations evidenced that the group nasally immunized with the combination of HBsAg, DT, Bp and TT (group 7) generated a greater and highly significant response compared to those induced in the group only immunized with TT, one of which had 0% seroconversion. The other groups nasally inoculated with TT in different antigenic mixtures had the same behavior. From this result, and bearing in mind the response of group 3 (containing HBsAg and TT), it is possible to state that HBsAg enhanced vaginal anti- TT IgA response at the same level as the other mixtures (Fig. 2B).

All groups inoculated by the intranasal route showed greater and highly significant responses compared to the group immunized with HB-DTP through intra-peritoneal injection. This group only had a 14 % seroconversion. Thus, it can be affirmed that not all antigens administered through the IP route give a vaginal response. It seems to be an exclusive property of certain antigens, including HBsAg.

### Vaginal anti- diphtheria toxoid response

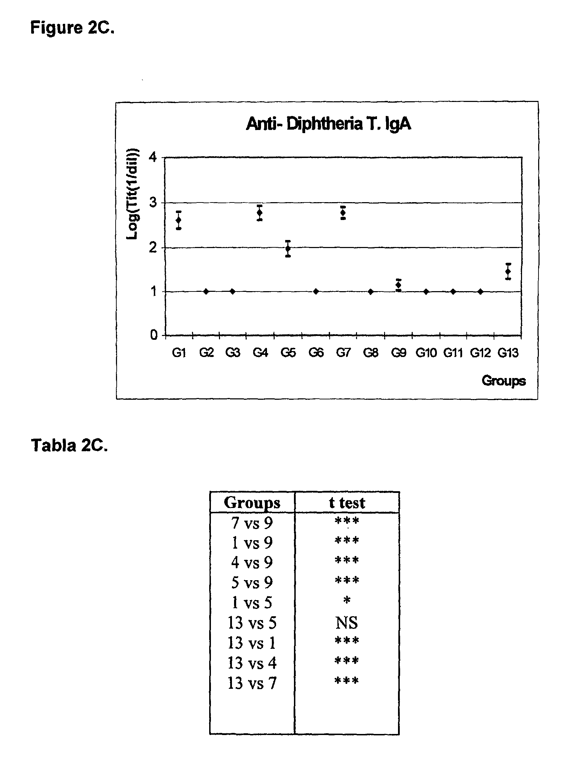

Anti-DT IgA response induced in the group immunized with the nasal tetravalent formulation as well as in the groups immunized with other nasal combinations containing DT were greater (p<0.001) than that generated in group 9 which was given a control preparation of DT in PBS (Fig. 2C).

Moreover, the response generated by the group given the four antigens nasally was also greater than that generated in the group of the intra-peritoneal-administered tetravalent vaccine; a similar behavior to that of TT.

In general, it was seen that the mucosally immunized groups developed vaginal IgA levels that were significantly higher than those of the group given the four antigens IP.

Anti- DT IgA levels belonging to group 1 did not differ from those obtained by the groups having higher immunogenicity, evidencing, as formerly confirmed for TT, that HBsAg induced a highly significant enhancement of the vaginal anti DT response compared to group 9, which was administered the toxoid in the PBS (Fig. 2C). It is possible, as we commented before, that this result may not have a direct benefit on the protection against tetanus and diphtheria. However, after evaluating the TT and DT models it can be suggested that the nasally administered combination of HBsAg with antigens of similar nature -not particulate but soluble- from other pathogens, free or anchored to these proteins, could generate an increase in the immune response at the vaginal level.

### Vaginal anti- Bordetella pertussis response

Vaginal anti- Bp response was divided into two levels, a lower one in which the group immunized with Bp in PBS was located, and another upper one corresponding to the immunized groups with all studied combinations. A greater and highly significant anti- Bp response was obtained by combined immunization (all containing HBsAg). Nevertheless, it should be noted that the response generated by Bp alone induced levels of nearly 1:100, unlike tetanus and diphtheria toxoid which scarcely seroconverted and only reached strong responses after their immunization in the antigen mixtures.

In the case of Bp the IP inoculation was not observed to generate any vaginal IgA responses. Therefore, it had a TT-like behavior, in which one generated by the IP route, a negligible IgA response in vaginal lavages. The other two antigens, HBsAg and DT alone generated weak but significant responses having 70 and 60 % of seroconversion respectively.

### Lung IgA responses

In the same way that vaginal responses are important in protecting against one of the pathogens, a response at the respiratory tract is very important for pathogens Bp and *Corynebacterium diphtheriae.* This does not exclude studies on anti-TT and anti-HBsAg responses for general knowledge about the level of mucosal immunologic activation after intranasal immunization with the tetravalent formulation of group 7.

### Lung anti- HBsAg responses

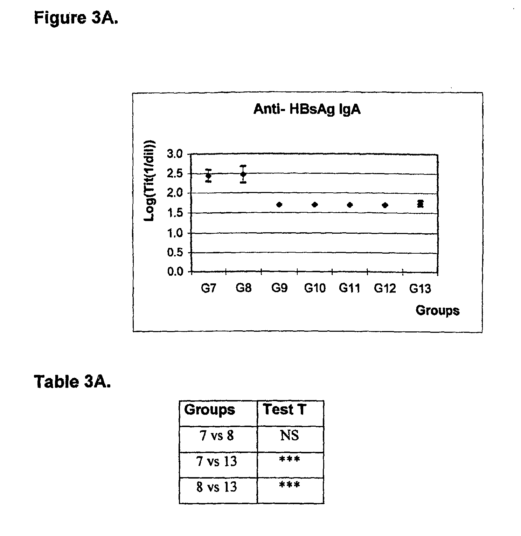

After determining the HBsAg-specific IgA response in lung lavages, it was concluded there were no significant differences between that generated by the group immunized nasally with the tetravalent combination (group 7) and that generated by the group which only received HBsAg by the same route (group 8). It is important to point out that during the evaluation of IgA levels in the lungs, there were similar serum IgG levels in groups immunized either with the mixture or with HBsAg in PBS. The strong boosting effect due to the fourth administration could trigger anti-HBsAg IgA levels in group 8. However the response was strong and not affected with that generated by HBsAg in PBS by the administration of a large amount of antigens (DT, TT and Bp) evidencing the high capacity of the route (Fig. 3A).

In the same figure it can be observed that the IP route, does not induce a significant response in lung lavages and the comparison with the intranasal groups widely favours the latter (p<0.001).

### Lung anti- tetanus toxoid responses

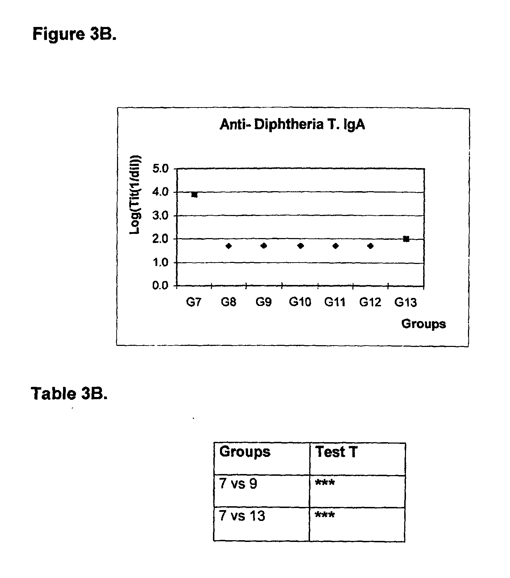

The anti-TT IgA response of the tetravalent nasal formulation was much higher (p<0.001) than that obtained with TT in PBS by the same route, or with intra-peritoneal administration together with HBsAg, DT, and Bp, in alum. The responses shown by these two latter control groups were almost negligible (Fig. 3B).

### Lung anti- diphtheria toxoid responses

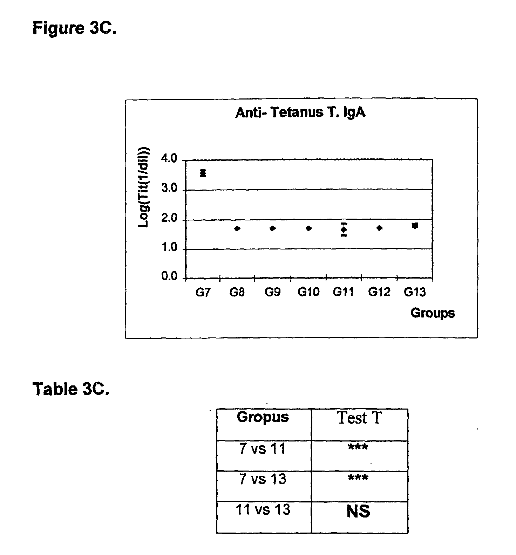

The combination of diphtheria toxoid with nasally administered HBsAg, TT and Bp, enormously enhanced its immunogenicity. This is based on the fact that results of the determinations of anti- DT IgA antibody responses indicated that there were highly significant differences (p<0.001) between the group immunized with the combination of the four antigens by the mucosal route (group 7) and groups 9 and 13, corresponding to DT in PBS nasally administered and the control of the tetravalent vaccine through the intra-peritoneal route, respectively. In the case of group 9, no mouse was positive to DT- specific IgA in a dilution of 1:100. In the IP group only one positive mouse was detected. Hence, this poor response strongly contrasts with that obtained by the tetravalent mixture, while there is also a contrast in the response obtained against TT in the homologous groups (Figs. 3C and 3B).

### Lung anti- Bordetella pertussis responses

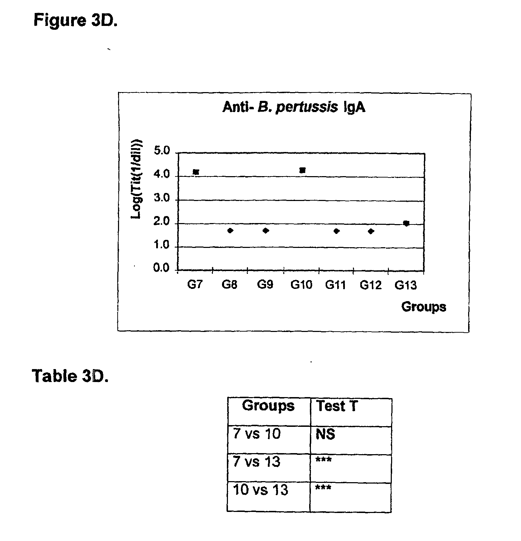

After comparing anti- Bp IgA responses developed in the group immunized with the tetravalent combination by the intranasal route (group 7) and the group exclusively immunized with inactivated Bp cells by the same route (group 10), no differences were found between them. However, when each response from these groups was compared to that generated by the group given the alum-adjuvanted mixture of four elements through the intra-peritoneal route, the superiority of both IgA responses was highly significant, demonstrating once again that only a mucosal inoculation favors strong increases in the IgA response detected in lung lavages (Fig. 3D).

Although the anti- Bp response in lung lavages was not enhanced with the mixture as in vaginal lavages, the increase in antigen number was not found to negatively affect immunogenicity. It should be noted that the anti- Bp response was very high in groups 7 and 10, with titers of a geometric mean of up to 10⁴. This result shows that Bp is an excellent immunogen by the intranasal route inducing lung IgA responses. The effect observed in antibody levels was of a maximum narrowing of intervals, evidencing their saturation. Since lung lavages were only performed as of group 7 and onward, group 2 was not compared to group 10 to study the effect of combining HBsAg and Bp, but we assume, considering titer levels and the characteristic of the response, the differences would be very small, if any.

### Example 3.

### Comparison of antibody response against the proteins FHA and pertussis toxin after nasal and systemic administrations of the formulations of groups 7 and 13 of example 1.

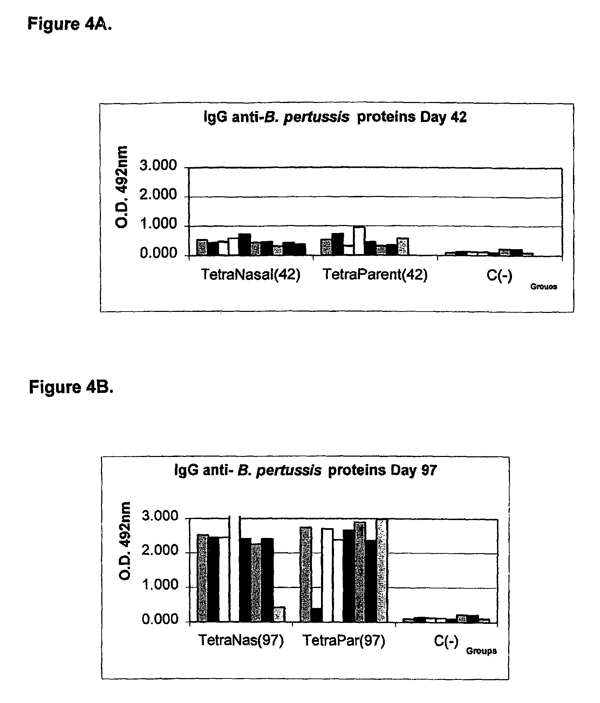

Because of previous reports mentioned in the specification suggest a lower ability of the intranasal route in order to elicit a response against the individual proteins of Bp: FHA and pertussis toxin, the evaluation of the response against them was carried out in groups 7 and 13, corresponding to the tetravalent formulation administered by the nasal and parenteral routes, respectively. This evaluation was achieved after three and four inoculations. The statistical analysis of the response of the evaluated bleedings demonstrated there were no significant differences between the nasal groups and the parenteral ones. Therefore, we could conclude that in the nasal tetravalent formulation the induction continues even after the inoculation of a 2.5 times lower amount of Bp (Fig. 4).

### Example 4.

### Lymphoproliferative response in the spleen after nasal administration.

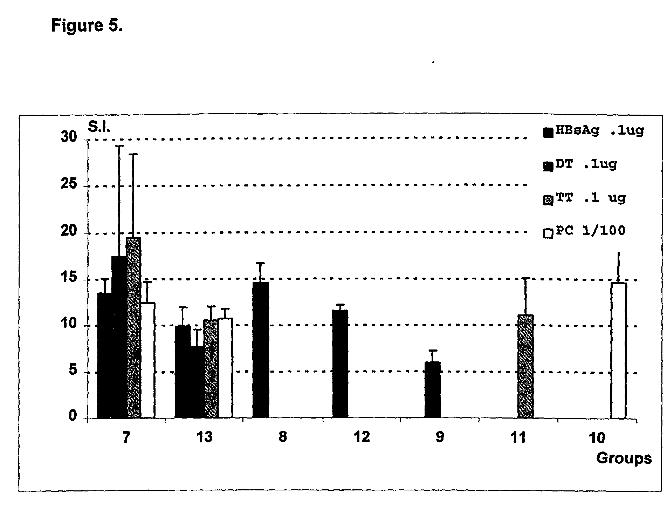

In order to study the spleen's proliferative response generated by the antigens of example 1, groups 7 to 13 were selected on day 100, extracting the spleen from at least four mice per group, making a lymphocytes pool, cultured in the presence of the antigens they had been immunized with. The results of the evaluation of the proliferative capacity of the antigens nasally administered in a tetravalent formulation are individually highlighted in the figure 5. Intra-peritoneal groups served as the control route.

As a result of this experiment it was evidenced that it is possible to obtain significant cellular response against all antigens present in the preparation, and even higher in some of them, from the nasal administration of multiple formulations of antigens, figure 5.

### Example 5

### HBsAg also act as an enhancer of the immunogenicity of the protein complex from the outer membrane vesicles of Neisseria meningifidis (OMPC).

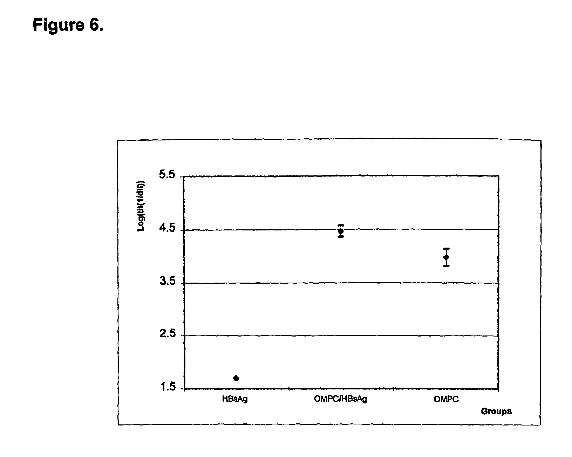

In order to explore whether HBsAg is able to enhance the response against co-administered antigens, an experiment was carried out in which groups of 8 BALB/c mice of 8 to 10 weeks of age were immunized with HBsAg, OMPC, and the corresponding control groups of the antigens alone. This experiment evidenced that the surface antigen was able to significantly enhance the immune response against OMPC and vice versa (Figure 6).

Other co-inoculated antigens received a similar effect on their immunogenicity because of the enhancing activity of the surface antigen. Some of them are inactivated virus, attenuated microorganisms and viral surface proteins, in addition to soluble proteins and bacterins.

### Example 6.

### Some true combinations. Potential and combination methods.

Some of the multiple combinations, which have demonstrated the immunogenicity of the individual components and which have generated an increased response against a high percentage of the antigens present within them are shown in the following table. They can be formulated as a whole in a liquid or lyophilized form for nasal administration.

| | | |
|---|---|---|
| 1- Hb - D | 6- Hb - PT | 11- Hb - DPT - Hib |
| 2- Hb - P | 7- Hb - DPT | 12- Hb - (IPV) |
| 3- Hb - T | 8- Hb - (Hib) | 13- Hb - DPT - Hib - (IPV) |
| 4- Hb - DP | 9- Hb - (OMPC) | |
| 5- Hb - DT | 10- Hb - (attenuated or inactivated virus, native or recombinant) | |

Among the antigenic combinations that could be mixed with HBsAg, antigens could be selected which could be formulated according to their application in age groups of: elderly persons, adolescents or newborn children, and according to the kind of mucosal disease. Included here are the antigens that enter through the mucosa, in which mucosal immunity is important. They can be selected according to the use of the antigen mixtures to prevent sexual, respiratory or mouth-intestinal diseases, according to the risk groups or to the travelers' needs, according to the organ (for instance: HBV, HCV and HAV), or according to the kind of disease (for instance: chronic sexually-transmitted diseases, viral sexually-transmitted diseases, etc...)

## Claims

1. A multivalent vaccine formulation for nasal administration containing hepatitis B virus surface antigen as a mucosal immunoenhancer of soluble antigens, bacterins and inactivated viruses.

2. A multivalent vaccine formulation for nasal administration according to claim 1, where one of the formulation antigens is the hepatitis B virus surface antigen itself.

3. A multivalent vaccine formulation for nasal administration according to claims 1 and 2 where together with the hepatitis B virus surface antigen a number n of other antigens are includedwhich receive an immunoenhancing effect due to their co-administration with HBsAg. Where n is of 1 to 20.

4. A multivalent vaccine formulation for nasal administration according to claims 1 to 3, where n comprises the tetanus toxoid antigen, which receives an immunoenhancing effect due to its co-administration with HBsAg.

5. A multivalent vaccine formulation for nasal administration according to claims 1 to 3, where n comprises the diphtheria toxoid antigen, which receives an immunoenhancing effect due to its co-administration with HBsAg.

6. A multivalent vaccine formulation for nasal administration according to claims 1 to 3, where n comprises a conjugate protein-polysaccharide corresponding to a vaccine antigen anti- *Haemophilus influenzae* type b, which receives an immunoenhancing effect due to its co-administration with HBsAg.

7. A multivalent vaccine formulation for nasal administration according to claims 1 to 3, where n comprises a conjugate protein-polysaccharide corresponding to polysaccharide C of *Neisseria meningitidis* conjugated to a carrier protein, which receives an immunoenhancing effect due to its co-administration with HBsAg.

8. A multivalent vaccine formulation for nasal administration according to claims 1 to 3, where n comprises a conjugate protein-polysaccharide, in which the polysaccharide part corresponds to a vaccine polysaccharide of *Pneumococcus pneumoniae*, which receives an immunoenhancing effect due to its co-administration with HBsAg.

9. A multivalent vaccine formulation for nasal administration according to claims 1 to 3, where n comprises inactivated microorganisms as vaccine antigens, which receive an immunoenhancing effect due to their co-administration with HBsAg.

10. A multivalent vaccine formulation for nasal administration according to claim 9, where a vaccine antigen may be the bacterin *Bordetella pertussis*, which receives an immunoenhancing effect because of it's co-administration with HBsAg.

11. A multivalent vaccine formulation for nasal administration according to claims 1 to 3, where n comprises inactivated virus as vaccine antigens, which receive an immunoenhancing effect because of their co-administration with HBsAg.

12. A multivalent vaccine formulation for nasal administration according to claims 1 to 3, where n comprises attenuated viruses as vaccine antigens, which receive an immunoenhancing effect because of their co-administration with HBsAg.

13. A multivalent vaccine formulation for nasal administration according to claims 1 to 3, where n comprises one or more of the antigens previously described in claims 4 to 12, or mixtures of them and other antigenic types, which receive an immunoenhancing effect because of their co-administration with HBsAg.

14. A multivalent vaccine formulation for nasal administration according to claims 1 to 13, where the volume of the final formulation is ranging from 50 microliters to 2 milliliters, depending on the size and the species to be immunized.

15. A multivalent vaccine formulation for nasal administration according to claims 1 to 14, where the amount of antigen to be inoculated range from 0.1 micrograms to 2 mg, depending on the kind of antigen and the species to be immunized.

16. A multivalent vaccine formulation for nasal administration according to claims 1 to 15, where the antigen mixture is dissolved in PBS, saline solution, water for injection or in any buffer solution used in medical practice or that allows the stability of the antigens.

17. A multivalent vaccine formulation for nasal administration according to claims 1 to 16, where the components are in a liquid or lyophilized state.

18. A multivalent vaccine formulation for nasal administration according to claims 1 to 17, where the administration is achieved with drops, a spray or pulverization.

19. A multivalent vaccine formulation for nasal administration according to claims 1 to 18, **characterized by** its use in humans or animals.

20. A multivalent vaccine formulation for nasal administration according to claims 1 to 19, **characterized by** its preventive or therapeutic use.
